# EUROPEAN PATENT SPECIFICATION

(11) **EP 1 492 477 B1**
(45) Date of publication and mention of the grant of the patent: **13.04.2016**
(21) Application number: 03709101.4
(22) Date of filing: 13.02.2003
(51) Int. Cl.: A61F 5/00, A61F 2/04

(54) **SATIATION DEVICES AND METHODS**
VORRICHTUNG UND VERFAHREN ZUR SÄTTIGUNG
METHODES ET DISPOSITIF POUR PROCURER UNE SENSATION DE SATIETE

(30) Priority: 08.04.2002 US 118289; 10.05.2002 US 379306 P; 16.01.2003 US 345914
(43) Date of publication of application: 05.01.2005
(60) Divisional application: 16153102.5
(73) Proprietor: Boston Scientific Scimed, Inc., Maple Grove, MN 55311-1566 (US)
(72) Inventor: STACK, Richard, S., Chapel Hill, NC 27514 (US); GLENN, Richard, A., Chapel Hill, NC 27516 (US); ATHAS, William, L., Durham, NC 27707 (US); WILLIAMS, Michael, S., Santa Rosa, CA 95404 (US); MOODY, Trevor, J., Seattle, WA 98115 (US); SILVERSTEIN, Fred, E., Seattle, WA 98112 (US); EVERY, Nathan, Seattle, WA 98105 (US)
(74) Representative: Vossius & Partner Patentanwälte Rechtsanwälte mbB
(86) International application number: PCT/US2003/004449
(87) International publication number: WO 2003/086247

(56) References cited:
- US-A- 4 899 747
- US-A- 5 306 300

## Description

### Field of the Invention

The present invention relates generally to the field of devices and methods for achieving weight loss in humans, and specifically to the use of devices implantable within the human stomach for controlling feelings of hunger and/or limiting food intake.

### Background of the Intention

Various medical approaches are used for controlling obesity. These approaches include diet, medication, and surgical procedures. One of the more successful surgical procedures is the vertical gastroplexy or proximal gastric pouch procedure in which a portion of the proximal stomach is formed into a small pouch with a small opening into the remainder of the stomach. This proximal gastric pouch may include a Roux-en-Y anastomosis in which a portion of the jejunum is connected to the pouch so as to shunt food from the proximal region of the stomach into the intestine, thereby minimizing absorption of food into the bloodstream. However, known complications are present with each of these procedures and more successful options are desired.

Berry (U.S. Patent No. 5,306,300) describes a tubular digestive screen which is placed in the lower stomach and threaded through the pylorus, the duodenum, the jejunem, and possibly into the ileum. The screen is intended to interfere with digestion, particularly of lipids.

Other alternatives include implantation of gastric balloons that prevent overeating by occupying volume within the stomach. Unfortunately, gastric balloons can migrate down the GI tract, causing obstruction and thus necessitating removal.

It is therefore desirable to provide a successful and minimally-invasive alternative to existing approaches for controlling obesity.

### Summary of the Intention

A satiation device utilizing principles of the present invention includes a sheath or liner positioned within the stomach. Food ingested by the patient passes through the sheath or liner, thereby minimizing contact between the ingested food and the stomach. It is believed that over time, reduced contact between food and the stomach will result in decreased Ghrelin production by the patient and a consequent decrease in appetite. In some embodiments, the satiation device may also include a proximal pouch and/or a distal bypass tube.

### Brief Description of the Drawings

Fig. 1 is a schematic illustration of a human stomach and a portion of the small intestine.
Fig. 2A is a side elevation view of a first embodiment of a satiation device.
Fig. 2B is a side elevation view of the pouch and chute of the embodiment of Fig. 2A.
Fig. 3 is a schematic illustration of a human stomach illustrating in vivo positioning of the embodiment of Fig. 2A.
Fig. 4 is a schematic illustration of a human stomach illustrating in vivo positioning of a second embodiment of a satiation device.
Fig. 5 is a schematic illustration of a human stomach showing in vivo positioning of an exclusion liner
Fig. 6 is a schematic illustration similar to Fig. 5 showing an exclusion liner having a distal tube.
Fig. 7 is a schematic illustration similar to Fig. 5 showing an exclusion liner having a proximal pouch.
Fig. 8 is a schematic illustration similar to Fig. 5 showing an exclusion liner having a proximal end at the middle stomach/proximal antrum.
Fig. 9 is a schematic illustration of a human stomach and proximal small intestine showing positioning of a bypass tube extending from the proximal stomach into the small intestine.
Fig. 10A is a cross-sectional side elevation view showing one example of a mechanism for attaching the pouch and the tube of Fig. 9.
Fig. 10B is a cross-sectional side elevation view showing a second example of a mechanism for attaching the pouch and the tube of Fig. 9.
Fig. 11 is a schematic illustration of a pylorus showing a tether connecting proximal and distal portions of the tube of Fig. 9 and extending past the ampulla vader.

### DETAILED DESCRIPTION

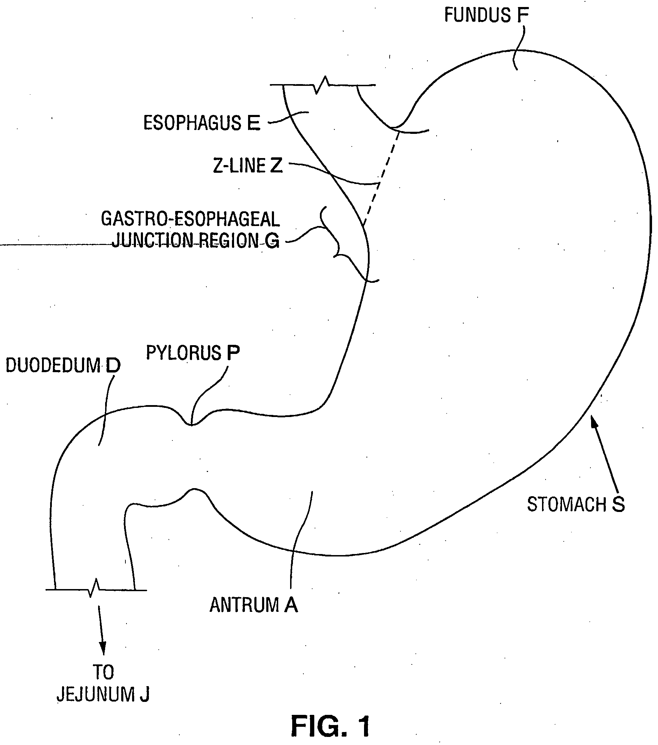

An anatomical view of a human stomach S and associated features is shown in Fig. 1. The esophagus E delivers food from the mouth to-the stomach S. The z-line or gastro-esophageal junction Z is the irregularly-shaped border between the thin tissue of the esophagus and the thicker tissue of the stomach wall. The gastro-esophageal junction region G is the region encompassing the distal portion of the esophagus E, the z-line, and the proximal portion of the stomach S.

Stomach S includes a fundus F at its proximal end and an antrum A at its distal end. Antrum A feeds into the pylorus P which attaches to the duodenum D, the proximal region of the small intestine. Within the pylorus P is a sphincter that prevents backflow of food from the duodenum D into the stomach. The middle region of the small intestine, positioned distally of the duodenum D, is the jejunum J.

Ghrelin is a satiety hormone secreted by cells in the stomach and the small intestine. Increased production of Ghrelin, such as before a meal, causes a person to experience hunger. After the person has eaten, production of Ghrelin decreases. It is presently believed that 85% of the Ghrelin-secreting cells are found in the stomach, and that the remaining 15% are in the antrum and small intestine. Obese patients have been found to possess significantly higher Ghrelin levels than non-obese patients. Moreover, recent studies have found that a patient's production of Ghrelin decreases significantly following gastric bypass procedures such as the Roux-en-y procedure described above. Various versions of the embodiments described herein provide an exclusion sleeve or liner situated within the stomach and extending from the proximal or middle stomach to the distal stomach or the small intestine. Over time, the presence of the exclusion sleeve will cause the Ghrelin-secreting cells to decrease Ghrelin production, causing the level of hunger experienced by a patient to decrease and thus resulting in weight loss by the patient.

Some of the embodiments of exclusion sleeves also include a pouch or funnel positioned at the gastro-esophageal junction region so as to form a small reservoir which collects masticated food from the esophagus. The pouch may limit the amount of food that can be consumed at one time. Additionally or alternatively, as the pouch fills with food, it may distend, imparting pressure against the upper stomach and lower esophageal sphincter causing the patient to experience sensations of fullness. Over time the food within this reservoir descends into the exclusion sleeve through a distal opening in the pouch. The pouch may optionally include a proximal tubular extension positionable within the esophagus to facilitate flow of food from the esophagus into the pouch. Various pouches of a type suitable for use with the exclusion sleeve are described in U.S. Application No. 10/118,289, filed April 8, 2002.

The devices may be modular in that where multiple components (e.g. a sleeve and pouch) are to be implanted, the various components may be provided separately from one another. In such a modular system, the separately implanted components may be attached to one another within the body during implantation, or certain ones of them may remain unattached to one another even after implantation. Alternatively, the physician may assemble the components to one another just prior to implantation. Modular components are desirable in that they permit the physician to select sizes for each component that are appropriate for the patient

Implantation of the described devices is preferably performed endoscopically, by passing the devices through the esophagus, preferably under endoscopic visualization. Alternatively, the devices may be implanted using surgical or laparoscopic procedures.

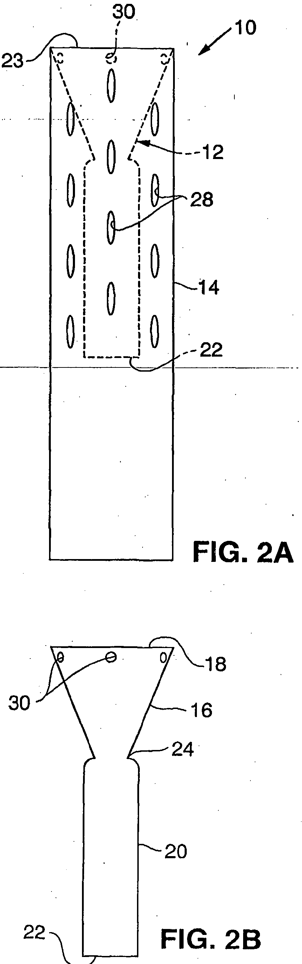

One embodiment of a satiation device is illustrated in Fig. 2A and includes a pouch 12 positioned within an elongate exclusion sleeve 14. For the purposes of this application, the term "satiation devices" will be used to mean devices intended to induce weight loss in one or more of a variety of ways. These include, but are not limited to, physically restricting the amount of food that can be consumed, and/or imparting pressure against portions of the body (e.g. stomach, esophagus, esophageal sphincter, etc) causing the patient to experience sensations of fullness, and/or affecting levels of hormones or other substances in the body that control or affect feelings of hunger, and/or affecting the amount of ingested food absorbed by the body.

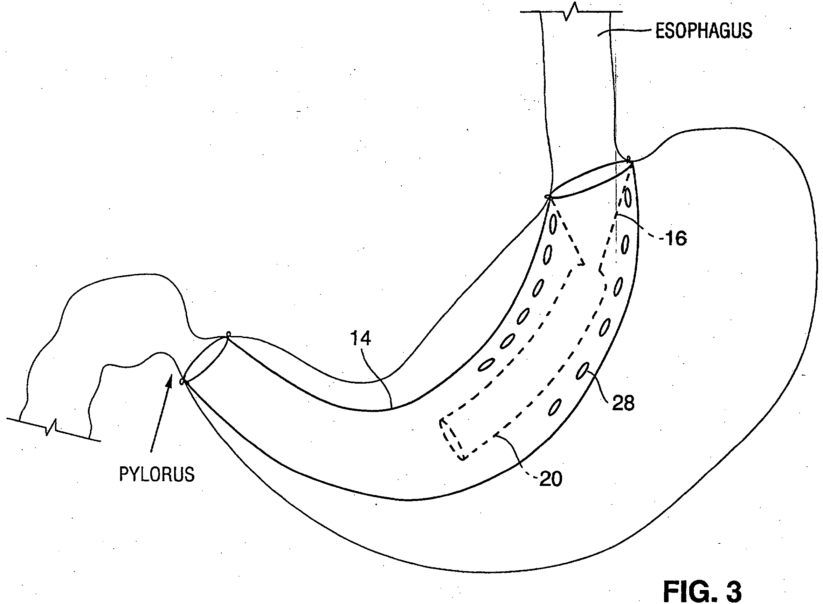

Pouch 12, shown without the sleeve in Fig. 2B, includes a funnel-shaped proximal portion 16 with an opening 18 that is positionable at the gastro-esophageal junction region (and preferably below the z-line) as shown in Fig. 3. Although a funnel shape is preferred here, a variety of alternative shapes may be used for the proximal portion of the pouch. For example, the pouch may have a much shorter proximal-to-distal dimension and thus take the shape of a shallow saucer with a small hole on its bottom surface. Other examples include, but are not limited to, egg shapes, other tapered shapes such as the shape of a "spinning top", cylindrical shapes, and other symmetrical or asymmetrical shapes.

Because of its small volume (which may be on the order of approximately 2 cc-300cc in volume, but is preferably in the range of 10-30 cc), the pouch functions to limit the amount of food that can be consumed at one time. Over time the food within this reservoir descends into the exclusion sleeve 14 through a distal opening in the pouch.

A distal chute 20 extends from the funnel-shaped proximal portion 16 and includes a distal opening 22. In this embodiment, the chute 20 tapers outwardly from the funnel 16 to form a valve 24 between the funnel and chute. The valve 24 may be formed of an elastic material that permits the valve opening to increase in size to permit large pieces of food to pass through. If desired, he diameter of the valve 24 may be adjustable by endoscopic means, such as by tightening a ligature around the valve, inflating an inflatable cuff positioned around the valve, or using various other means. This increases the effectiveness of the pouch by decreasing the exit diameter - thereby causing food to exit the pouch more slowly and prolonging the feeling of fullness experienced by the patient. Likewise, the diameter of the valve 24 may be endoscopically increased by deflating a fluid filled restrictive cuff, using a tool to cut or stretch open the valve, or using various other means, to increase the ability of the patient to tolerate the pouch by permitting more rapid emptying of the pouch.

Pouch 12 may be formed of a flexible material that will prevent passage of food through the sides of the pouch. Examples of such materials include, but are not limited to polyesters (e.g. Dacron® polyester), ePTFE fabric (e.g. GoreTex® fabric or others), a polyurethane such as ChronoFlex® polyurethane, nylon fabrics, silicone, other polymeric materials, and bio-absorbable materials (e.g. PLLA, PGA, PCL, poly-amhydride etc). The pouch may be formed of a composite of compliant, semi-compliant and/or non-compliant materials which give different regions of the pouch different degrees of compliance so as to allow/limit expansion of the pouch in various locations. For example, it may be desirable to provide the pouch with a fairly elastic exit port to as to prevent occlusion in the event a large piece of food is ingested and/or to control the exit pressure of food from the pouch, whereas the proximal end of the pouch may be stiffer to prevent bulging. Varying degrees of compliance may also be built into the pouch by varying the cross-sectional thickness in different regions of the pouch. The material may be coated with a lubricious, bio-compatible, chemically inert material, such as paraleyne, to reduce friction on the base material's surface which will help prevent sticking and food build up on the device.

Sleeve 14 is a flexible tube having a proximal opening 23 and a distal opening 26. The sleeve material may be similar to the material described with respect to the pouch 12, but should be sufficiently flexible to permit peristalsis. The exterior of the sleeve material may be impregnated with substances known to inhibit release of Ghrelin or other hormones associated with feelings of satiety. Such substances may be chemical or pharmaceutical substances, therapeutic molecules or cells, or genetic material. The sleeve may also be impregnated with an acid to inhibit production of Gastrin, or its exterior may be impregnated with an anti-Gastrin antibody.

The sleeve 14 and pouch 12 are preferably attached to one another at their respective proximal openings 18, 23 using sutures, clips, adhesives or other suitable means. These components maybe attached to one another during manufacture or just prior to implantation, or they may be separately implanted.

The proximal portion of the sleeve 14 contains a plurality of openings 28 sized to allow gastric secretions to enter the sleeve 14 to aid in digesting food that has passed from the pouch 12 into the sleeve 14 and to allow drainage of the secretions from the stomach. The stomach secretions exit the stomach via the sleeve and drain into the pylorus. The openings 28 may take the form of perforations or slots in the sleeve wall. Alternatively, the openings may take the form of apertures in mesh or porous regions in a portion of the sleeve. For example, ePTFE with an open cell structure is a material that can be constructed to allow gastric secretions to enter without allowing food to exit.

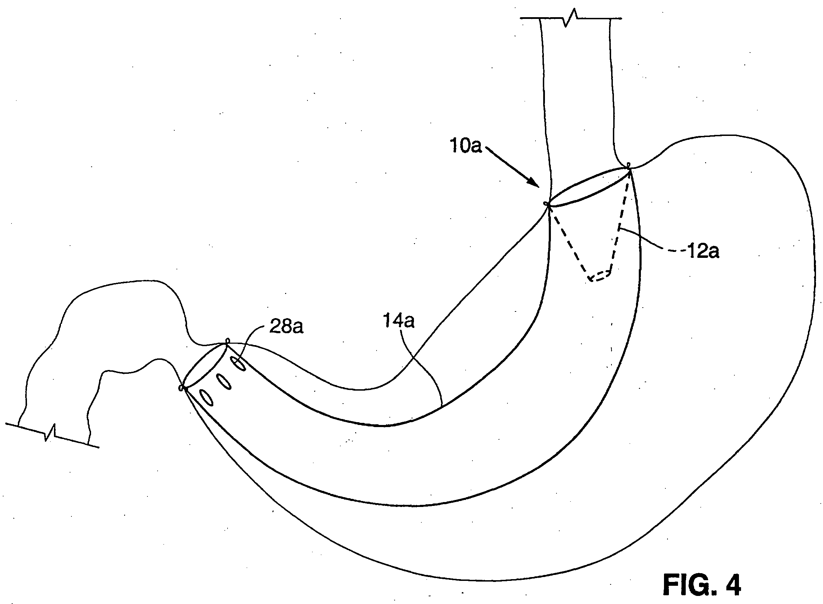

In the embodiment shown in Fig. 3, the openings are positioned such that when the pouch 12 is positioned within the sleeve 14 for use, the distal opening 22 of the chute 20 is preferably distal to the openings 28 so as to prevent food from exiting the sleeve through the openings 28. The openings 28 may be positioned in any other locations as well, particularly if the openings are small enough to prevent food from passing through. Examples of alternative locations for the openings include locations near the distal end of the sleeve (see Fig. 4), or locations substantially along the entire sleeve length.

The diameter of the sleeve 14 is sufficiently large to allow the pouch to be enclosed within the sleeve 14, but is preferably sufficiently narrow to permit acids produced within the stomach to flow into contact with the walls of the antrum. It is believed that such contact is needed for normal regulation of the hormone Gastrin. Gastrin is a physiological regulator of gastric acid secretion within the stomach. Increased Gastrin levels result in increased secretion of gastric acids. Acid levels that are too high can produce ulcers.

Secretion of Gastrin (and thus resultant secretion of gastric acids) is inhibited when cells in the antrum detect a low pH. It is thus important for acids in the stomach to contact the antrum to ensure normal regulation of Gastrin. If the acids are not permitted to contact the antrum, Gastrin production would increase, and might thus cause an increased production of gastric acids that could lead to stomach ulcers.

Methods for implanting satiation devices such as pouch 12 are shown and described in U.S. Application No. 10/118,289 filed April 8, 2002. For example, some of the disclosed methods involve packaging the satiation device within a deployment tube, inserting the distal end of the tube into the stomach (preferably from the esophagus), ejecting the satiation device from the tube using a pushrod passed through tube, and then securing the satiation device using sutures, clips, adhesives, radial forces, stents or stent-like structures, etc. Such methods may be utilized to deploy the satiation devices described herein, including the pouch 12 and sleeve 14.

During implantation the pouch 12 is secured at the gastro-esophageal junction region G using sutures, clips, adhesives, stents or stent-like structures, or other suitable means. One suture attachment device found useful for applying sutures between the pouch and tissue is the "Sew-Right" suturing device available from LSI Solutions of Victor, New York.

Although the pouch may be secured to the esophageal tissue, it is more preferable to apply sutures/clips below the Z-line to allow for attachment to the thicker tissue of the stomach wall. Suture attachment points, which may take the form of holes, anchor loops, eyelets, windows, or grommets 30 in the pouch may be used to provide regions (which may be reinforced) for anchoring the sutures. Although as few or as many of such suture/clip attachment points as needed may be used, at least four such points are desirable, such as at 90° intervals around the pouch, so as to enable the pouch to be secured around the full circumference of the tissue. The suture attachment points may be made of a suitably dense radio-opaque material, such as titanium or gold, to add in visualization of the device during or after the procedure. Each suture attachment point may also be marked using a different color to facilitate identification and orientation of sutures. If the pouch is formed of a less durable material, the proximal portion of the pouch (in which the eyelets 30 are located) may be formed of more durable material such as a woven material, Dacron ® polyester or ePTFE fabric so as to provide a more secure sewing region. Although loops, grommets, eyelets or reinforced regions may be advantageous, the pouch may alternatively be provided without suture attachment points formed of special materials (with or without identifying markings) - in which case the sutures are passed directly through the pouch material.

The flexible pouch and/or sleeve material may be reinforced with, constructed of, or supported by supporting members, such as a soft mesh, a cage structure, ribs, rings etc. The supporting members may be formed of stainless steel, polymer, shape memory materials such as nitinol, shape memory alloys, or shape memory polymers, or thickened regions of material. The pouch-and/or sleeve may be constructed so as to be self-expanding, such that the pouch and/or sleeve springs radially open into an expanded condition upon ejection from a deployment device or catheter as described above.

The proximal end of sleeve 14, near proximal opening 23, may be attached to the pouch 12 alone, or it may be attached to the pouch 12 and to the surrounding tissue. The sleeve 14 is attached at its distal opening to the distal stomach (e.g. at the antrum) near the pylorus, so as to allow food exiting the sleeve 14 to flow out of the stomach to the small intestine. Attachment is made using sutures, clips, adhesives, stents or stent-like structures or other suitable means.

Fig. 4 shows an alternative embodiment of a satiation device 10a using a pouch 12a and sleeve 14a. The satiation device 10a differs from that of Fig. 3 primarily in that the pouch 12a is provided without a distal chute (see chute 20 Fig. 3), and in that the openings 28a in the sleeve are positioned at the distal end of the sleeve so as to permit drainage of gastric secretions into the sleeve 28a and from the sleeve into the pylorus. Drainage of gastric secretions is desirable to avoid accumulation of such secretions in the stomach.

The sleeve 14a (and also the sleeve 14 of Fig. 3) may be semi-impermeable, allowing gastric secretions to enter without allowing food to exit. Materials such as ePTFE with open-cell structure (e.g. node to fibril-lengths of 20 - 100 micron) are suitable for this purpose.

The embodiments of Figs. 3 and 4 are believed to cause weight loss in a number of ways. First, as the pouch fills with food, it may distend, imparting pressure against the upper stomach and lower esophageal sphincter causing the patient to experience sensations of fullness after consuming small quantities of food. Second, it is believed that isolating consumed food from the walls of the stomach using the exclusion sleeve 14, 14a will lead to a temporary increase in the patient's production of Ghrelin followed by a "burn out" phenomenon over time in which the Ghrelin is reduced, in turn leading to decreased sensations of hunger. Third, digestion is delayed and absorption of the food is minimized.

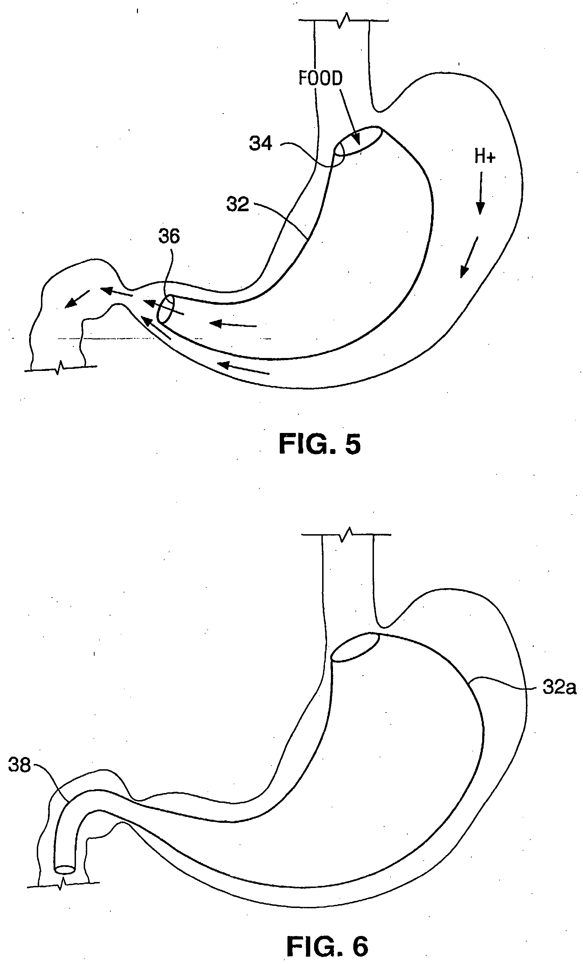

Figs. 5 through 8 show additional embodiments that also control satiety by isolating consumed food from the walls of the stomach so as to eventually decrease day-to-day production of Ghrelin by the patient due to the above described "burn out" phenomenon. Each of these embodiments includes an exclusion liner that is positioned within the stomach such that consumed food passes through the liner and then exits the liner to flow into the small intestine via the pylorus.

Referring to Fig. 5, a first exclusion liner 32 includes a proximal opening 34 positioned in the proximal stomach, such as at the gastro-esophageal junction region G, and a distal opening 36 positioned in the antrum A, preferably adjacent to the pylorus P. The liner 32 maybe proportioned to substantially line the stomach while still allowing clearance for gastric acids (labeled H+ in Fig. 5) to contact the walls of the antrum surrounding the liner - so as to prevent overproduction of Gastrin as described above. Alternatively, the liner may have a significantly narrower diameter if desired. As shown in Fig. 5, liner 32 reduces contact between ingested food and the stomach, including the fundus and the antrum.

The proximal region adjacent to the proximal opening 34 is preferably secured to tissue at the gastro-esophageal junction region below the Z line using sutures, clips, adhesives, stents or stent-like structures or other suitable means. The distal region adjacent to the distal opening may be secured in a leak-proof manner to the distal antrum, or it may be secured more loosely to permit gastric secretions to exit the stomach into the pylorus (see arrows in Fig. 5). For example, clips or sutures, etc, may be applied in a manner that leaves a gap between the distal opening 36 and the pylorus to permit drainage of gastric secretions. Alternatively, if such drainage is desired, the liner 32 may be provided with a plurality of openings (see, for example, openings 28a in Fig. 4) that allow gastric secretions to flow into the liner and then into the pylorus. As another alternative, liner may be supported by a cage structure having resilient cage members that contact the walls of the stomach to prevent migration of the liner within the stomach, but that allow clearance between the stomach walls and the exterior of the liner. Cage structures of a type that may be adapted for this purpose are shown and described in U.S. Application No. 09/940,110, filed August 27, 2001.

Fig. 6 shows an exclusion liner 32a that is similar to the liner 32 of Fig. 5), but that further includes a distal tube 38 that is secured in the pylorus or duodenum using sutures, anchors, clips, stents or stent-like structures, adhesives, etc. Again, this distal connection may be leak proof to prevent drainage of secretions, or it may configured to allow such drainage.

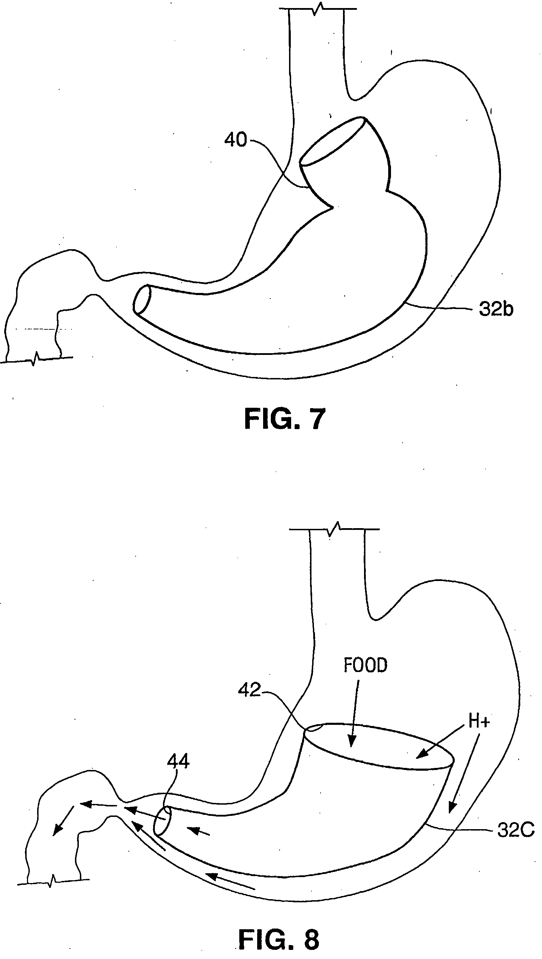

Fig. 7 shows an exclusion liner 32b that is similar to the liner of Fig. 5, except that it includes a proximal pouch 40 attachable at the gastro-esophageal junction region. As with the embodiment of Figs. 3 and 4, the pouch 40 fills with food when the patient eats, causing the patient to experience sensations of fullness after consuming small quantities of food. The pouch 40 may be integral with the liner 32b, or it may be separately attachable prior to or during implantation. As with the embodiments of Fig. 5 and Fig. 6, the distal end of the liner 32b may be attached to the distal antrum in a manner that permits drainage of secretions present outside the liner, or in a manner that occludes such drainage. The embodiment of Fig. 7 may also be provided with a distal tube similar to the distal tube 38 of the Fig. 6 embodiment.

Fig. 8 shows an antral exclusion liner 32c proportioned to extend from the middle stomach or the proximal antrum to the distal antrum. Liner 32c includes a large proximal opening 42 and a smaller distal opening 44 as shown. The proximal region of the liner 32c is secured to the surrounding walls of the stomach, and the distal region is secured to the distal antrum. Liner 32c may be secured in a manner that permits flow of some food and stomach secretions around the liner 32c as indicated by arrows in Fig. 8, or it may be secured tightly against the stomach walls such that all food and stomach secretions are directed through the liner 32c. The embodiment of Fig. 8 may be provided with a distal -tube similar to the tube 38 of Fig. 6.

Materials that may be used for the liners of Figs. 5 - 8 include flexible materials that will prevent passage of food through the sides of the pouch. The materials may be fluid impermeable or slightly permeable. Slightly permeable materials (e.g. ePTFE with open-cell structure on the order of 20 -100 micron node to fibril length) may be desirable in that they allow gastric secretions to pass into the liner without allowing food to exit.

Examples of materials useful for the liner include, but are not limited to polyesters (e.g. Dacron® polyester), ePTFE fabric (e.g. GoreTex® fabric or others), a polyurethane such as ChronoFlex® polyurethane, nylon fabrics, silicone, other polymeric materials, and bio absorbable materials (e.g. PLLA, PGA, PCL, poly-amhydride etc). The liners may be formed of a composite of compliant, semi-compliant and/or non-compliant materials which give different regions of the sleeve/pouch different degrees of compliance so as to allow/limit expansion of the sleeve/pouch in various locations. For example, it may be desirable to provide the liner with a fairly elastic exit port to as to prevent occlusion in the event a large piece of food is ingested. The material may be coated with a lubricious, bio-compatible, chemically inert material, such as paraleyne, to reduce friction on the base.

The exterior of the liner material may be impregnated with substances known to inhibit release of Ghrelin or other hormones associated with feelings of satiety. Such substances may be chemical or pharmaceutical substances, therapeutic molecules or cells, or genetic material. The liner may also be impregnated with an acid to inhibit production of Gastrin, or its exterior may be impregnated with an anti-Gastrin antibody, or any of a variety of therapeutic drugs or molecules.

The liner may be reinforced with, constructed of, or supported by a supporting structure, such as a soft mesh, coil, a cage structure, ribs, rings etc. The supporting structure may be formed of stainless steel, polymer, shape memory materials (such as nitinol, shape memory alloys, or shape memory polymers), bio-absorbable materials or, in the case of a silicone liner, thickened regions of silicone. The supporting structure may be located at the interior or exterior of the liner material. It may be molded into or sewn to the liner material, or it may be attached using a suitable adhesive. If a tightly woven mesh or tightly wound coil is provided, the flexible material may be eliminated. Alternatively, a mesh may be provided having a polymeric material embedded in the interstices of the mesh, in which case a separate internal or external covering of liner material may be eliminated. The polymer may be impregnated with an agent that will decrease Ghrelin secretion or neutralize stomach acidity.

The inner diameter of the liner (and/or supporting structure) may be coated with lubricious material such as Teflon or parylene to ease the passage of food through the liner.

The liner is preferably constructed so as to be self-expanding, such that the pouch springs radially open into an expanded condition upon ejection from a deployment device or catheter. In one example of a method of deploying a liner such as the liners of Figs. 5-8, the liner may be compressed and inserted into a deployment tube. In this example, the distal end of the deployment tube is inserted (preferably through the esophagus) into the stomach and the liner ejected from the tube using a pushrod passed through tube. The liner expands within the stomach, and the physician secures the liner to the stomach using sutures, clips, adhesives, stents or stent-like structures, radial forces, etc.

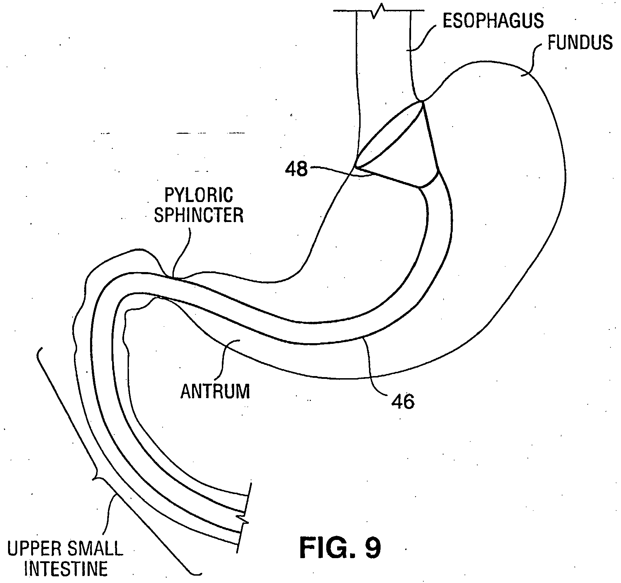

Fig. 9 illustrates an alternate satiation device that includes an elongate bypass tube 46 that is implanted to extend from the proximal stomach, through the pylorus and into the small intestine (for example, through the first 24 inches of the small intestine). As with prior embodiment, tube 46 reduces the amount of contact between ingested food and the stomach and thus may eventually result in decreased Ghrelin production. It also functions similar to a Roux-en-y bypass in that it reduces the amount of surface of the small intestine that is available for absorbing food. The tube is preferably made of a thin-walled polymer that is flexible enough to allow peristalsis within the small intestine. It also bypasses the bile and pancreatic ducts, which insert digestive enzymes into the small intestine.

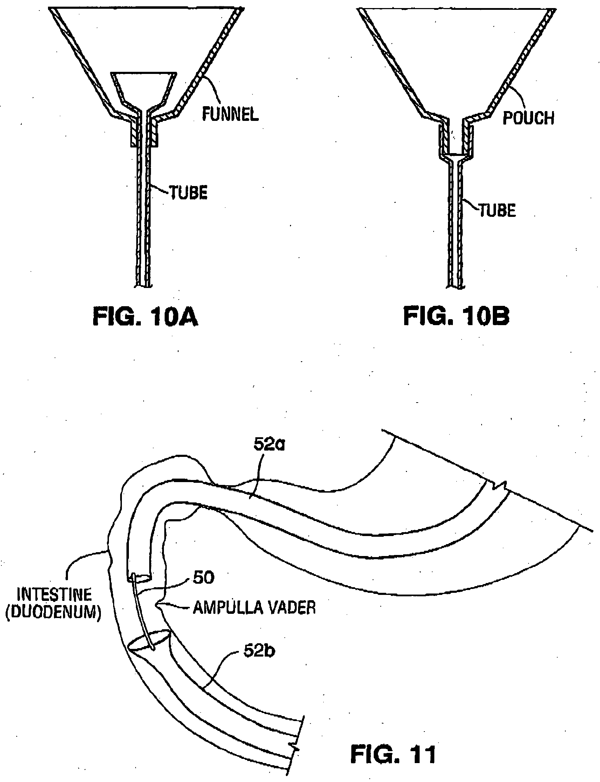

It may be desirable to position the tube so that it does not contact the ampulla of vader (an opening in the duodenum through which bile passes into the duodenum) so as to minimize the chance of irritation and choleocystitus. For example, a narrow tether 50 (Fig. 11) may connect the proximal portion 52a of the tube (which is disposed proximal of the ampulla vader) to the distal portion 52b of the tube so as to avoid obstructing the ampulla. Distal portion 52b may include a funnel-shaped proximal end to facilitate re-entry of food into the tube 46 after the food has passed the ampulla vader.

Lastly, referring again to Fig. 9, tube 46 may be provided with a proximal pouch 48, similar to the pouch of Fig. 3, that may distend when filled with ingested food, imparting pressure against the upper stomach and lower esophageal sphincter causing the patient to experience sensations of fullness. The pouch 48 and tube 46 may be separately implantable and then attached to one another in situ.

Figs. 10A and 10B show two examples of configurations for attaching the pouch 48 and tube 46. In one configuration, the proximal end of the tube 48 maybe inserted into the distal opening of the pouch 46 as shown in Fig. 10A. In another, the distal end of the pouch 46 may be inserted into the proximal end of the tube 48 as shown in Fig 10B. Naturally, other means of attachment may be used, including sutures, hooks, stents or stent-like structures and/or adhesivesr

Various embodiments of satiation devices have been described herein. These embodiments are given by way of example and are not intended to limit the scope of the present invention. It should be appreciated, moreover, that the various features of the embodiments that have been described may be combined in various ways to produce numerous additional embodiments. Moreover, while various materials, dimensions, shapes, implantation locations, etc. have been described for use with disclosed embodiments, others besides those disclosed may be utilized without exceeding the scope of the invention.

The method not forming part of the invention defined by the following numbered paragraphs have also been described hereinbefore.
1. A method for inducing weight loss in a patient having a stomach, an antrum, and a pylorus, the method comprising the steps of:
   positioning a prosthesis within a patient's stomach, the prosthesis having a --proximal opening and a distal opening and a passage extending between the proximal and distal openings;
   causing the patient to ingest food material, at least a portion of the food material passing through the proximal opening into the prosthesis;
   allowing food material to exit the prosthesis and to flow into the pylorus; and
   allowing gastric secretions of the stomach to flow around at least a portion of the prosthesis and into the pylorus.
2. The method of paragraph 1, wherein the causing and allowing steps reduce the amount of food material absorbed by an interior surface of the stomach.
3. The method of paragraph 1, further including the steps of repeating the causing and allowing steps multiple times, resulting in a decrease of Ghrelin secretion by the stomach and thereby reducing sensations of hunger in the patient.
4. The method of paragraph 1, wherein the causing step reduces contact between ingested food material and an interior surface of the antrum.
5. The method of paragraph 1, wherein the stomach includes a fundus and wherein the causing step reduces contact between ingested food material and an interior surface of the fundus.
6. The method of paragraph 1, wherein the step of allowing gastric secretions to flow into the pylorus includes allowing the gastric secretions to flow from the stomach into the prosthesis.
7. The method of paragraph 6, wherein the gastric secretions flow into the prosthesis through an opening in the proximal portion of the prosthesis.
8. The method of paragraph 6, wherein the gastric secretions flow into the prosthesis through one or more openings in a wall of the prosthesis.
9. The method of paragraph 8, wherein the one or more openings are proportioned to permit flow of gastric secretions into the prosthesis while substantially preventing ingested food material from passing through the one or more openings.
10. The method of paragraph 1, wherein at least a portion of the gastric secretions flow into the pylorus without passing through the prosthesis.
11. The method of paragraph 1, wherein the positioning step includes securing a proximal portion of the prosthesis within the gastro-esophageal junction region such that the proximal opening receives food directly from the esophagus.
12. The method of paragraph 11, wherein the securing step includes securing the proximal portion to tissue below the Z-line of the gastro-esophageal junction region.
13. The method of paragraph 1, wherein the positioning step includes securing the distal portion of the prosthesis within the distal portion of the stomach.
14. The method of paragraph 1, wherein the prosthesis is expandable from a collapsed position in which the prosthesis has a first diameter to an expanded position in which the prosthesis has a second, larger, diameter, and wherein the positioning step includes the steps of:
   with the prosthesis in the collapsed position, inserting the prosthesis into the stomach; and
   expanding the prosthesis from the collapsed to the expanded position.
15. The method of paragraph 14, wherein the prosthesis self-expands to the expanded position.
16. The method of paragraph 1, wherein the proximal opening of the prosthesis is smaller than the distal opening.
17. The method of paragraph 1, further including the steps of:
   positioning a tubular pouch in the gastro-esophageal junction region of the stomach, the pouch including a proximal opening and a distal opening;
   causing food ingested by the patient to pass from the esophagus into the proximal opening of the pouch, and to pass from the pouch through the distal opening and into the tubular prosthesis.
18. The method of paragraph 17, where the proximal opening of the pouch is smaller than the distal opening.
19. The method of paragraph 17, wherein the pouch is tapered from a proximal portion of the pouch to a more distal portion of the pouch.
20. The method of paragraph 18, wherein a distal portion of the pouch includes an elongate chute, and wherein the distal opening is in the chute.
21. The method of paragraph 20, wherein a one-way valve is provided between the chute and the pouch, and wherein the method includes preventing flow of food material from the chute into the pouch.
22. The method of paragraph 17, wherein food material passing into the pouch causes the pouch to distend and impart pressure against a portion of the stomach, causing the patient to experience sensations of fullness.
23. The method of paragraph 17, wherein the positioning step includes securing the pouch to tissue below the Z-line of the gastro-esophageal junction region.
24. The method of paragraph 17, wherein at least a portion of the pouch is disposed within the prosthesis.
25. The method of paragraph 17, wherein the prosthesis extends from a distal portion of the pouch.
26. The method of paragraph 1, wherein the positioning step positions the proximal opening of the prosthesis at a position between a fundus of the stomach and the pylorus
27. The method of paragraph 1, wherein the positioning step positions the proximal opening of the prosthesis adjacent to the esophagus and positions the distal opening of the prosthesis adjacent to the pyloric sphincter.
28. The method of paragraph 1, wherein the prosthesis includes a distal tube, and wherein the positioning step includes positioning the distal tube through the pylorus, and wherein the method includes causing food material to pass from the prosthesis into the distal tube.
29. The method of paragraph 1, wherein the distal tube includes a first portion, a second portion, and a tether connecting the first and second portions, and wherein the positioning step includes positioning the distal tube such that the tether is adjacent the ampulla vader.

## Claims

1. An apparatus for inducing weight loss in a patient, the apparatus comprising:
a prosthesis (14) having a proximal opening (23) and a distal opening and a passage between the proximal and distal openings, at least a portion of the prosthesis positionable within a patient's stomach, and securing means for securing the proximal portion of the prosthesis at the gastro-esophageal junction region, such that food from the esophagus passes directly into the proximal opening,
wherein the proximal opening of the prosthesis is proportioned to receive at least a portion of mod material ingested by the patient, and the distal opening is proportioned to permit said food material to exit the prosthesis and to flow into the pylorus or into the small intestine,
and the prosthesis is configured to permit gastric secretions of the stomach to flow around at least a portion of the prosthesis and into the pylorus.

2. The apparatus of claim 1, wherein the prosthesis includes a wall formed of a material that prevents passage of food material through the wall.

3. The apparatus of claim 2, wherein at least a portion of the wall is fluid permeable to permit gastric secretions to pass through the sidewall and into the prosthesis.

4. The apparatus of claim 1, wherein the prosthesis is positionable in the stomach to permit a least a portion of gastric secretions of the stomach to flow into the pylorus without passing through the prosthesis.

5. The apparatus of claim 1, wherein the distal opening is proportioned to permit said food material to exit the prosthesis and to flow into the pylorus.

6. The apparatus of claim 1, wherein the securing means include sutures.

7. The apparatus of claim 1, wherein the securing means include clips.

8. The apparatus of claim 1, wherein the securing means include adhesives.

9. The apparatus of claim 1, wherein the securing means includes a structure expandable from a streamlined positioned for insertion into the stomach to an expanded position for contact with the walls of the stomach.

10. The apparatus of claim 1, further including securing means for securing the distal portion of the prosthesis within the stomach.

11. The apparatus of claim 10, wherein the securing means include clips.

12. The apparatus of claim 10, wherein the securing means include adhesives.

13. The apparatus of claim 10, wherein the securing means includes a structure expandable from a streamlined positioned for insertion into the stomach to an expanded position for contact with the walls of the stomach.

14. The apparatus of claim 1, further including a pouch (12), the pouch including a proximal opening (18) and a distal opening (22), the pouch positionable in the gastro-esophageal junction region of the stomach such that food ingested by the patient passes from the esophagus into the proximal opening of the pouch, and from the pouch through the distal opening and into the prosthesis.

15. The apparatus of claim 14, wherein the proximal opening of the pouch is smaller than the distal opening of the pouch.

16. The apparatus of claim 15, wherein the pouch is tapered from a proximal portion of the pouch to a more distal portion of the pouch.

17. The apparatus of claim 15, wherein a distal portion of the pouch includes an elongate chute (20), and wherein the distal opening (22) is in the chute.

18. The apparatus of claim 17, further including a one-way valve (24) between the chute and the pouch.

19. The apparatus of claim 14, wherein the pouch is formed of an expendable material such that food material passing into the pouch causes the pouch to distend and impart pressure against a portion of the stomach, causing the patient to experience sensations of fullness.

20. The apparatus of claim 14, wherein at least a portion of the pouch is disposed within the prosthesis.

21. The apparatus of claim 14, wherein the prosthesis extends from a distal portion of the pouch.

22. The apparatus of claim 1, wherein the prosthesis includes a tail section (46) proportioned to extend through the pylorus and into the small intestine.

23. The apparatus of claim 1, wherein the tail section includes a pair of tubes (52a, 52b) and a tether (50) extending between the tubes.

24. The apparatus of claim 1, wherein the distal opening is smaller than the proximal opening.

25. The apparatus of claim 1, wherein the prosthesis is proportioned to reduce contact between ingested food material and an interior surface of the antrum.

26. The apparatus of claim 1, wherein the prosthesis is proportioned to reduce contact between ingested food material and an interior surface of the fundus.

## Patentansprüche

1. Vorrichtung zum Induzieren von Gewichtsverlust bei einem Patient, wobei die Vorrichtung aufweist:
eine Prothese (14) mit einer proximalen Öffnung (23) und einer distalen Öffnung sowie einem Durchgang zwischen der proximalen und distalen Öffnung, wobei mindestens ein Abschnitt der Prothese im Magen eines Patienten positionierbar ist,
und einer Befestigungseinrichtung zum Befestigen des proximalen Abschnitts der Prothese am gastroösophagealen Übergangsbereich, so dass Nahrung aus der Speiseröhre direkt in die proximale Öffnung passiert,
wobei die proximale Öffnung der Prothese so proportioniert ist, dass sie mindestens einen Anteil von Nahrungsmaterial empfängt, das vom Patient aufgenommen wird, und die distale Öffnung so proportioniert ist, dass das Nahrungsmaterial die Prothese verlassen und in den Magenausgang oder in den Dünndarm fließen kann, und die Prothese so konfiguriert ist, dass Magensekretionen des Magens um mindestens einen Abschnitt der Prothese und in den Magenausgang fließen können.

2. Vorrichtung nach Anspruch 1, wobei die Prothese eine Wand aufweist, die aus einem Material gebildet ist, das Durchgang von Nahrungsmaterial durch die Wand verhindert.

3. Vorrichtung nach Anspruch 2, wobei mindestens ein Abschnitt der Wand fluiddurchlässig ist, damit Magensekretionen durch die Seitenwand und in die Prothese passieren können.

4. Vorrichtung nach Anspruch 1, wobei die Prothese im Magen so positionierbar ist, dass mindestens ein Anteil von Magensekretionen des Magens in den Magenausgang fließen kann, ohne die Prothese zu durchlaufen.

5. Vorrichtung nach Anspruch 1, wobei die distale Öffnung so.proportioniert ist, dass das Nahrungsmaterial die Prothese verlassen und in den Magenausgang fließen kann.

6. Vorrichtung nach Anspruch 1, wobei die Befestigungseinrichtung Nähte aufweist.

7. Vorrichtung nach Anspruch 1, wobei die Befestigungseinrichtung Clips aufweist.

8. Vorrichtung nach Anspruch 1, wobei die Befestigungseinrichtung Kleber aufweist.

9. Vorrichtung nach Anspruch 1, wobei die Befestigungseinrichtung eine Struktur aufweist, die aus einer strömungsgünstigen Position zur Einführung in den Magen in eine expandierte Position zum Kontakt mit den Wänden des Magens expandierbar ist.

10. Vorrichtung nach Anspruch 1, ferner mit einer Befestigungseinrichtung zum Befestigen des distalen Abschnitts der Prothese im Magen.

11. Vorrichtung nach Anspruch 10, wobei die Befestigungseinrichtung Clips aufweist.

12. Vorrichtung nach Anspruch 10, wobei die Befestigungseinrichtung Kleber aufweist.

13. Vorrichtung nach Anspruch 10, wobei die Befestigungseinrichtung eine Struktur aufweist, die aus einer strömungsgünstigen Position zur Einführung in den Magen in eine expandierte Position zum Kontakt mit den Wänden des Magens expandierbar ist.

14. Vorrichtung nach Anspruch 1, ferner mit einem Beutel (12), wobei der Beutel eine proximale Öffnung (18) und eine distale Öffnung (22) aufweist und der Beutel im gastroösophagealen Übergangsbereich des Magens so positionierbar ist, dass vom Patient aufgenommene Nahrung aus der Speiseröhre in die proximale Öffnung des Beutels und aus dem Beutel durch die distale Öffnung und in die Prothese passiert.

15. Vorrichtung nach Anspruch 14, wobei die proximale Öffnung des Beutels kleiner als die distale Öffnung des Beutels ist.

16. Vorrichtung nach Anspruch 15, wobei der Beutel von einem proximalen Abschnitt des Beutels zu einem distaleren Abschnitt des Beutels zuläuft.

17. Vorrichtung nach Anspruch 15, wobei ein distaler Abschnitt des Beutels eine längliche Rutsche (20) aufweist und wobei die distale Öffnung (22) in der Rutsche liegt.

18. Vorrichtung nach Anspruch 17, ferner mit einem Einwegventil (24) zwischen der Rutsche und dem Beutel.

19. Vorrichtung nach Anspruch 14, wobei der Beutel aus einem expandierbaren Material gebildet ist, so dass in den Beutel passierendes Nahrungsmaterial bewirkt, dass sich der Beutel aufweitet und Druck auf einen Abschnitt des Magens ausübt, wodurch der Patient ein Völlegefühl empfindet.

20. Vorrichtung nach Anspruch 14, wobei mindestens ein Abschnitt des Beutels in der Prothese angeordnet ist.

21. Vorrichtung nach Anspruch 14, wobei sich die Prothese von einem distalen Abschnitt des Beutels erstreckt.

22. Vorrichtung nach Anspruch 1, wobei die Prothese ein hinteres Teilstück (46) aufweist, das so proportioniert ist, dass es sich durch den Magenausgang und in den Dünndarm erstreckt.

23. Vorrichtung nach Anspruch 1, wobei das hintere Teilstück ein Paar Schläuche (52a, 52b) und eine Leine (50) aufweist, die sich zwischen den Schläuchen erstreckt.

24. Vorrichtung nach Anspruch 1, wobei die distale Öffnung kleiner als die proximale Öffnung ist.

25. Vorrichtung nach Anspruch 1, wobei die Prothese so proportioniert ist, dass sie Kontakt zwischen aufgenommenem Nahrungsmaterial und einer Innenfläche des Antrums reduziert.

26. Vorrichtung nach Anspruch 1, wobei die Prothese so proportioniert ist, dass sie Kontakt zwischen aufgenommenem Nahrungsmaterial und einer Innenfläche des Fundus reduziert.

## Revendications

1. Appareil pour induire une perte de poids chez un patient, l'appareil comprenant :
une prothèse (14) ayant une ouverture proximale (23) et une ouverture distale et un passage entre les ouvertures proximale et distale, au moins une partie de la prothèse pouvant être positionnée à l'intérieur de l'estomac d'un patient, et des moyens de fixation pour fixer la partie proximale de la prothèse au niveau de la région de jonction gastro-oesophagienne, de sorte que la nourriture provenant de l'oesophage passe directement dans l'ouverture proximale,
dans lequel l'ouverture proximale de la prothèse est proportionnée pour recevoir au moins une partie de matière alimentaire ingérée par le patient, et l'ouverture distale est proportionnée pour permettre à ladite matière alimentaire de sortir de la prothèse et de s'écouler dans le pylore ou dans l'intestin grêle,
et la prothèse est configurée pour permettre aux sécrétions gastriques de l'estomac de s'écouler autour d'au moins une partie de la prothèse et dans le pylore.

2. Appareil selon la revendication 1, dans lequel la prothèse comprend une paroi formée avec un matériau qui empêche le passage de matière alimentaire à travers la paroi.

3. Appareil selon la revendication 2, dans lequel au moins une partie de la paroi est perméable au fluide pour permettre aux sécrétions gastriques de passer à travers la paroi latérale et dans la prothèse.

4. Appareil selon la revendication 1, dans lequel la prothèse peut être positionnée dans l'estomac pour permettre à au moins une partie des sécrétions gastriques de l'estomac de s'écouler dans le pylore sans passer à travers la prothèse.

5. Appareil selon la revendication 1, dans lequel l'ouverture distale est proportionnée pour permettre à ladite matière alimentaire de sortir de la prothèse et de s'écouler dans le pylore.

6. Appareil selon la revendication 1, dans lequel les moyens de fixation comprennent des sutures.

7. Appareil selon la revendication 1, dans lequel les moyens de fixation comprennent des agrafes.

8. Appareil selon la revendication 1, dans lequel les moyens de fixation comprennent des adhésifs.

9. Appareil selon la revendication 1, dans lequel les moyens de fixation comprennent une structure expansible d'une position aérodynamique pour l'insertion dans l'estomac à une position expansée pour le contact avec les parois de l'estomac.

10. Appareil selon la revendication 1, comprenant en outre des moyens de fixation pour fixer la partie distale de la prothèse à l'intérieur de l'estomac.

11. Appareil selon la revendication 10, dans lequel les moyens de fixation comprennent des agrafes.

12. Appareil selon la revendication 10, dans lequel les moyens de fixation comprennent des adhésifs.

13. Appareil selon la revendication 10, dans lequel les moyens de fixation comprennent une structure expansible d'une position aérodynamique pour l'insertion dans l'estomac à une position expansée pour le contact avec les parois de l'estomac.

14. Appareil selon la revendication 1, comprenant en outre une poche (12), la poche comprenant une ouverture proximale (18) et une ouverture distale (22), la poche pouvant être positionnée dans la région de jonction gastro-oesophagienne de l'estomac de sorte que la nourriture ingérée par le patient passe de l'oesophage dans l'ouverture proximale de la poche, et de la poche à travers l'ouverture distale et dans la prothèse.

15. Appareil selon la revendication 14, dans lequel l'ouverture proximale de la poche est inférieure à l'ouverture distale de la poche.

16. Appareil selon la revendication 15, dans lequel la poche est progressivement rétrécie d'une partie proximale de la poche à une partie plus distale de la poche.

17. Appareil selon la revendication 15, dans lequel la partie distale de la poche comprend une goulotte allongée (20) et dans lequel l'ouverture distale (22) est dans la goulotte.

18. Appareil selon la revendication 17, comprenant en outre une valve à une voie (24) entre la goulotte et la poche.

19. Appareil selon la revendication 14, dans lequel la poche est formée avec un matériau expansible de sorte que la matière alimentaire passant dans la poche amène la poche à se détendre et à communiquer la pression contre une partie de l'estomac, amenant le patient à ressentir des sensations de satiété.

20. Appareil selon la revendication 14, dans lequel au moins une partie de la poche est disposée à l'intérieur de la prothèse.

21. Appareil selon la revendication 14, dans lequel la prothèse s'étend à partir d'une partie distale de la poche.

22. Appareil selon la revendication 1, dans lequel la prothèse comprend une section de queue (46) proportionnée pour s'étendre à travers le pylore et dans l'intestin grêle.

23. Appareil selon la revendication 1, dans lequel la section de queue comprend une paire de tubes (52a, 52b) et une attache (50) s'étendant entre les tubes.

24. Appareil selon la revendication 1, dans lequel l'ouverture distale est plus petite que l'ouverture proximale.

25. Appareil selon la revendication 1, dans lequel la prothèse est proportionnée pour réduire le contact entre la matière alimentaire ingérée et la surface intérieure de l'antre.

26. Appareil selon la revendication 1, dans lequel la prothèse est proportionnée pour réduire le contact entre la matière alimentaire ingérée et la surface intérieure du fond.
